# EUROPÄISCHE PATENTANMELDUNG

(11) **EP 4 760 324 A1**
(43) Veröffentlichungstag der Anmeldung: **17.06.2026**
(21) Anmeldenummer: 24220218.2
(22) Anmeldetag: 16.12.2024
(51) Int. Cl.: G01R 33/28, A61B 6/46, A61B 5/00

(54) **VERFAHREN ZUM ERKENNEN EINES WARNHINWEISES EINES PATIENTEN WÄHREND DER PATIENT MITTELS EINES BILDGEBUNGSSYSTEMS UNTERSUCHT WIRD, SOWIE PATIENTENWARNEINRICHTUNG UND BILDGEBUNGSSYSTEM**

(71) Anmelder: Siemens Healthineers AG, 91301 Forchheim (DE)
(72) Erfinder: HÖCHT, Philipp, 91207 Lauf (DE); WOLF, Felix, 91088 Bubenreuth (DE); ROAS-LÖFFLER, Michael, 91052 Erlangen (DE); HOFMANN, Christian, 91054 Erlangen (DE); SCHNELL, Wilfried, 91301 Forchheim (DE)
(74) Vertreter: Siemens Healthineers Patent Attorneys

(57) **Zusammenfassung**

Die Erfindung betrifft ein Verfahren zum Erkennen eines Warnhinweises eines Patienten (8), während der Patient (8) mittels eines Bildgebungssystems (1) untersucht wird, wobei
- zumindest ein akustisches Warnsignal (15), mit welchem der Warnhinweis des Patienten (8) repräsentiert wird, auf Basis einer Betätigung eines Bestätigungselements (13) durch den Patienten (8) erzeugt und in eine Umgebung (17) des Bildgebungssystems (1) ausgesendet wird,
- das in die Umgebung (17) ausgesendete akustische Warnsignal (15) empfangen und einer elektronischen Auswerteeinheit (20) bereitgestellt wird,
- das empfangene und bereitgestellte akustische Warnsignal (15) durch die elektronische Auswerteeinheit (20) ausgewertet wird, und
- der Warnhinweis des Patienten (8) auf Basis des ausgewerteten akustischen Warnsignals (15) erkannt wird.

Des Weiteren betrifft die Erfindung eine Patientenwarneinrichtung (12) sowie ein Bildgebungssystem (1).

## Beschreibung

Unabhängig vom grammatikalischen Geschlecht eines bestimmten Begriffes sind Personen mit männlicher, weiblicher oder anderer Geschlechteridentität mit umfasst.

Die vorliegende Erfindung betrifft ein Verfahren zum Erkennen eines Warnhinweises eines Patienten während der Patient mittels eines Bildgebungssystems untersucht wird.

Des Weiteren betrifft die Erfindung eine Patientenwarneinrichtung für ein Bildgebungssystem.

Ebenfalls betrifft die Erfindung ein Bildgebungssystem mit einer Bildgebungsmodalität und einer Patientenwarneinrichtung.

Bei einer Untersuchung eines Patienten mittels eines Bildgebungssystems, wie beispielsweise eines Magnetresonanztomografiesystems (MRT), muss dem Patienten die Möglichkeit gegeben werden, einem Bediener, also einem Bedienpersonal des Bildgebungssystems, ein Warnsignal übermitteln zu können. Dies ist daher erforderlich, da sich der Patient während des Untersuchungsvorgangs, bei welchem sich der Patient beispielsweise im Tunnel des MRT-Systems befindet, unwohl oder sogar klaustrophobisch fühlen kann. Beispielsweise wird diese Anforderung laut der Norm "60601-2-33:2022" definiert beziehungsweise vorgegeben.

In bisherigen MRT-Systemen kommen üblicherweise pneumatische Elemente, wie ein pneumatischer Quetschball, zum Einsatz. Dieser ist über einen langen Schlauch mit einem pneumatisch-elektrischen Wandler, insbesondere am Fußende des Patiententisches, verbunden. Dies hat jedoch den Nachteil, dass vor allem der lange Schlauch während des Vorbereitungsvorgangs hinsichtlich des Patientenuntersuchungsvorgangs umständlich ist. Dieser Schlauch kann sich verheddern und aufgrund seiner Steifigkeit ist dieser schwer handzuhaben und kann beim Bewegen des Patiententisches eingeklemmt werden. Vor allem kann der Schlauch für den Patienten und insbesondere für das Bedienpersonal als Stolperstelle gefährlich im Untersuchungsraum herumliegen. Vor und nach Untersuchungen ist des Weiteren das Verstauen beziehungsweise das Aufräumen des Schlauches umständlich.

Daher besteht eine Aufgabe der vorliegenden Erfindung darin, die Handhabung einer Patientenwarneinrichtung, mit welcher ein Patient eine Warnung an ein Bedienpersonal bereitstellen kann, einfacher für das Bedienpersonal zu gestalten.

Diese Aufgabe wird durch ein Verfahren, eine Patientenwarneinrichtung sowie ein Bildgebungssystem gemäß den unabhängigen Patentansprüchen gelöst. Sinnvolle Weiterbildungen ergeben sich aus den abhängigen Patentansprüchen.

Ein Aspekt der Erfindung betrifft ein Verfahren zum Erkennen eines Warnhinweises eines Patienten während der Patient mittels eines Bildgebungssystems untersucht wird, wobei
- zumindest ein akustisches Warnsignal, mit welchem der Warnhinweis des Patienten repräsentiert wird, auf Basis einer Betätigung eines Bestätigungselements durch den Patienten erzeugt und in eine Umgebung des Bildgebungssystems ausgesendet wird,
- das in die Umgebung ausgesendete akustische Warnsignal empfangen und einer elektronischen Auswerteeinheit bereitgestellt wird,
- das empfangene und bereitgestellte akustische Warnsignal durch die elektronische Auswerteeinheit ausgewertet wird, und
- der Warnhinweis des Patienten auf Basis des ausgewerteten akustischen Warnsignals erkannt wird.

Durch das vorgeschlagene Verfahren kann eine einfachere und insbesondere verbesserte Möglichkeit einer Auslösung einer Warnung oder eines Warnhinweises während eines Untersuchungsvorgangs für einen Patienten bereitgestellt werden. Entgegen den bisher verwendeten pneumatischen Systemen werden hier nun keinerlei Schläuche oder kabelgebundene Verbindungen hinsichtlich der Pneumatik mehr benötigt. Somit kann hier die Warnmöglichkeit für den Patienten auf eine einfache, bauraumeinsparende und insbesondere kostengünstige Weise realisiert werden. Vor allem kann aufgrund der minimalen Infrastrukturanforderungen eine kostengünstigere Lösung im Gegensatz zu den pneumatischen Systemen geschaffen werden. Dies kann durch das vorgeschlagene Verfahren dadurch erreicht werden, dass die Aktivierung der Warnung, also eines Notrufes des Patienten, auf einer akustischen Signalverarbeitung beruht. Durch das Betätigen löst der Patient das Erzeugen des akustischen Warnsignals aus.

Mit dem akustischen Warnsignal kann der Warnhinweis, also die Warnung beziehungsweise der Notruf, des Patienten repräsentiert werden. Das Betätigungselement kann beispielsweise als kompaktes tragbares Bauteil, wie ein in der Hand tragbares Bauteil, ausgebildet sein. Da eine akustische Signalübermittlung verwendet wird, entfallen insbesondere im Vergleich zum Stand der Technik die pneumatischen Schläuche oder sonstige entsprechende Verbindungen. Durch Auswertung dieses akustischen Warnsignals kann systemseitig, insbesondere mithilfe einer Software, eine signaltechnische Auswertung vorgenommen werden. Anhand dieser Auswertung kann der Warnhinweis des Patienten systemseitig erkannt werden. Somit kann hier auch mittels einer einfachen und unkomplizierten Methode festgestellt werden, ob während eines Untersuchungsvorgangs am Patienten Probleme aufgetreten sind. Somit kann dem Patienten schnellstmöglich Hilfe bereitgestellt werden.

Insbesondere kann es sich bei dem Bildgebungssystem um ein MRT-System (Magnetresonanztomografiesystem) handeln. Hierbei befindet sich der Patient in einem Patiententunnel, also in einer Röhre des MRT-Systems. Ein solches MRT-System befindet sich während des Untersuchungsvorgangs in einem Untersuchungsraum, welcher wiederum abgeschirmt ist. Das Bedienpersonal befindet sich hierzu in einem separaten Raum, wodurch keine direkte Sicht zwischen dem Bedienpersonal und dem Patienten vorliegen kann. Somit ist das Erkennen des Warnhinweises des Patienten, wenn sich der Patient beispielsweise unwohl fühlt, essentiell. Durch das signaltechnische Erzeugen und Aussenden eines akustischen Warnsignals kann dies problemlos empfangen und vor allem schnell ausgewertet werden, sodass das Bedienpersonal über den Warnhinweis des Patienten schnellstmöglich informiert werden kann.

In einem Ausführungsbeispiel ist vorgesehen, dass auf Basis des erkannten Warnhinweises eine Warnung an ein Bedienpersonal des Bildgebungssystems ausgesendet wird und auf Basis des erkannten Warnhinweises eine Sicherheitsfunktion des Bildgebungssystems automatisch aktiviert wird. Der Patient kann aufgrund einer aktuellen Notsituation den Warnhinweis ausgeben, sodass hier durch den Patienten bewusst eine Warnung beziehungsweise eine Hilfsanforderung initiiert wird. Durch diese signaltechnische Warnung kann ohne große Zeitverzögerung der Warnhinweis und somit die Warnung des Patienten erkannt werden. Sollte dies der Fall sein, kann eine entsprechende Warnung an das Bedienpersonal ausgegeben werden. Dies kann beispielsweise akustisch, optisch und/oder haptisch erfolgen. Somit kann das Bedienpersonal sich über den aktuellen Zustand des Patienten informieren.

In einem Ausführungsbeispiel ist vorgesehen, dass das zumindest eine akustische Warnsignal so erzeugt wird, dass das akustische Warnsignal in einem Hörfrequenzbereich, also im Bereich zwischen 20 Hz und 20 kHz, oder in einem Ultraschallfrequenzbereich, also in einem Frequenzbereich größer 20 kHz, liegt. Je nach Anwendungsfall und/oder nach Ausgestaltung des Bildgebungssystems kann vor allem der Frequenzbereich hinsichtlich des akustischen Warnsignals variiert werden. Bei der Ausgestaltung des akustischen Warnsignals im Ultraschallfrequenzbereich ergibt sich der Vorteil, dass dieses im Vergleich zu dem Hörfrequenzbereich nicht durch den Patienten störend wahrgenommen wird.

Wenn das akustische Warnsignal im hörbaren Bereich ausgestaltet wird, so kann es unter Umständen zu Interferenzen kommen. Beispielsweise kann die Frequenz des akustischen Signals bei 20 kHz definiert werden. Bei dieser beispielhaften Frequenz kann ein um etwa 60 dB niedriger Lärmpegel erreicht werden. Weitere Frequenzbereiche, insbesondere Frequenzbereiche des Hörfrequenzbereichs oder des Ultraschallbereichs sind ebenfalls denkbar. Eine im Ultraschallfrequenzbereich höhere Frequenz bietet beispielsweise den Vorteil, dass ein besseres SNR (Signalrauschverhältnis) bezüglich des Gradientenlärms vorliegen kann. Bei der Verwendung des Ultraschallfrequenzbereichs ist zu beachten, dass das Ultraschallsignal aufgrund von Körperspulen des Bildgebungssystems oder anderen Teilen, wie Patientendecken, die den Ultraschallsensor beispielsweise abdeckt, abgeschwächt werden kann. Daher wäre ein Frequenzbereich ein Kompromiss aus erreichbarer Worst-Case-Signalstärke und maximalem Lärm.

Beispielsweise kann das akustische Warnsignal mit einer fest vorgegebenen Frequenz im Ultraschallbereich ausgesendet werden. Dies kann in der Auswerteeinheit als Information gespeichert sein, so dass bei Detektion eines akustischen Signals mit dieser Frequenz eine einfache Erkennung hinsichtlich des Warnhinweises vorgenommen werden kann. Somit kann hier eine einfache Signaldetektion und somit eine Kosteneinsparung erreicht werden.

In einem Ausführungsbeispiel ist vorgesehen, dass zumindest eine Komponente des akustischen Signals derart erzeugt wird, indem zumindest ein durch das Bildgebungssystem hervorgerufenes Störsignal berücksichtigt wird. Da bei Untersuchungsvorgängen eines Bildgebungssystems insbesondere elektromagnetische Felder auf Basis von Spulen erzeugt werden können, können verschiedenste Störsignale beim Untersuchungsvorgang auftreten. Damit es hinsichtlich des akustischen Signals zu keinen Interferenzen oder sonstigen sich gegenseitig negativ auswirkenden Erscheinungen kommen kann, ist je nach Einsatzgebiet, also je nach Anwendungssituation, der Frequenzbereich für das akustische Warnsignal vorzugeben. Somit kann je nach Ausgestaltung des Bildgebungssystems ein hierzu vorzunehmender Frequenzbereich hinsichtlich des akustischen Signals vorgegeben werden. Somit kann erreicht werden, dass eine effiziente Erkennung oder Detektion des Warnhinweises auf Basis des akustischen Signals durchgeführt werden kann.

In einem Ausführungsbeispiel ist vorgesehen, dass das akustische Signal auf Basis von einer Identifizierungsinformation oder auf Basis von einer Zusatzinformation moduliert wird. Um insbesondere Fehldetektionen vermeiden zu können, ist eine effiziente und insbesondere genaue Erkennung des Warnhinweises erforderlich. Hierbei kann das akustische Signal moduliert werden, so dass das akustische Signal bei der Erkennung beziehungsweise Detektion eindeutig im Hinblick auf einen durch den Patienten ausgelösten Warnhinweis identifizierbar ist. Hierbei kann das akustische Signal derart moduliert werden, indem die Frequenz, die Amplitude und die Phase entsprechend angepasst wird. Hierbei können die verschiedensten Modulationsverfahren angewendet werden. Für die Modulation können beispielsweise Pulsamplitudenmodulationsverfahren oder Korrelationssequenz-Verfahren angewendet werden. Hier können auf Basis von gewünschten Modulationseigenschaften verschiedenste Parameter des akustischen Signals moduliert werden. Auf Basis der durchgeführten Modulation kann eine Empfangseinheit das akustische Signal robust beziehungsweise zuverlässig, erkennen und insbesondere identifizieren.

Mit der Zusatzinformation können zusätzliche Informationen der Patientenwarneinrichtung, wie ein aktueller Ladezustand einer Batterie, mittels des akustischen Warnsignals zusätzlich übertragen werden.

In einem Ausführungsbeispiel ist vorgesehen, dass neben dem zumindest einen akustischen Signal zumindest ein weiteres akustisches Signal, mit welchem der Warnhinweis ebenfalls repräsentiert wird, erzeugt und ausgesendet wird, wobei das zumindest eine akustische Signal und das zumindest eine weitere akustische Signal zueinander verschiedene Frequenzen aufweisen, und wobei das zumindest eine weitere akustische Signal empfangen und ebenfalls der elektronischen Auswerteinheit bereitgestellt wird.

Durch das Verwenden von zwei oder mehr akustischen Signalen, bei welchen es sich um Ultraschallsignale handeln kann, kann eine robustere Identifizierung beziehungsweise Detektion beziehungsweise Erkennung hinsichtlich des tatsächlichen Warnhinweises des Patienten durchgeführt werden. Durch das Aussenden und Auswerten von zwei (oder mehr) akustischen Signalen kann eine robustere und weniger fehleranfällige Erkennung vorgenommen werden. Speziell kann dadurch verhindert werden, dass ein anderweitig vorhandenes Stör-Signal erfasst und als akustisches Signal hinsichtlich des Warnhinweises interpretiert wird. Durch das Aussenden und Empfangen der beiden akustischen Signale, welche unterschiedliche Frequenzen aufweisen, kann eine verbesserte Erkennung vorgenommen werden. Beispielsweise kann erst dann von einer Gefahrenlage des Patienten systemseitig ausgegangen werden, wenn beide Signale entsprechend erkannt und ausgewertet werden konnten. Somit können Fehlauslösungen beziehungsweise unbeabsichtigte Warnsituationen vermieden werden. Des Weiteren wäre ebenso möglich, dass mehr als zwei akustische Signale verwendet werden. Beispielsweise können fünf akustische Signale ausgesendet werden und wenn mindestens drei dieser fünf akustischen Signale detektiert wurden, kann der Warnhinweis an den Bediener ausgegeben werden.

Beispielsweise kann das Betätigungselement derart ausgebildet sein, dass der Patient eine gewisse vorgesehene Betätigung durchführen muss, um beide akustischen Warnsignale erzeugen und aussenden zu können. Somit kann beispielsweise verhindert werden, dass bei einer kurzen oder zu leichten Betätigung eine fehlerhafte beziehungsweise unbeabsichtigte Warnung vorgenommen wird. Erst wenn beide Signale ausgesendet und entsprechend empfangen und ausgewertet werden konnten, kann eine sichere Notsituation des Patienten erkannt und entsprechende Maßnahmen eingeleitet werden.

Des Weiteren ist denkbar, dass weitere akustische Signale verwendet werden, so dass mehrere Signale, welche zueinander verschiedene Frequenzen aufweisen können, ausgesendet werden. Somit können Fehlalarme beziehungsweise fehlerhafte Erkennungsvorgänge vermieden werden. Wenn mehrere akustische Signale ausgesendet werden, können spezielle Protokolle verwendet werden, um eine Fehlererkennung und gegebenenfalls eine Fehlerkorrektur durchführen zu können. Beispielsweise können mehrere Ultraschallsignale ausgesendet werden und mittels Demodulation entsprechend ausgewertet werden.

Ein weiterer Aspekt der Erfindung betrifft eine Patientenwarneinrichtung für ein Bildgebungssystem, mit
- einer Signalerzeugungseinheit, welche ausgebildet ist, zumindest ein akustisches Warnsignal auf Basis einer Betätigung eines Bestätigungselements der Patientenwarneinrichtung durch einen Patienten zu erzeugen und in eine Umgebung des Bildgebungssystems auszusenden,
- einer Signalerfassungseinheit, welche ausgebildet ist, das in die Umgebung ausgesendete akustische Warnsignal zu empfangen und einer elektronischen Auswerteeinheit der Patientenwarneinrichtung bereitzustellen,
- der elektronischen Auswerteeinheit, welche ausgebildet ist, das empfangene und bereitgestellte akustische Warnsignal bezüglich eines durch den Patienten während eines mit dem Bildgebungssystem am Patienten durchgeführten Untersuchungsvorgangs initiierten Warnhinweises auszuwerten und den Warnhinweis des Patienten auf Basis des ausgewerteten akustischen Warnsignals zu erkennen.

Insbesondere kann mit der soeben geschilderten Patientenwarneinrichtung ein Verfahren nach dem vorhergehenden Aspekt oder der vorteilhaften Weiterbildung davon ausgeführt beziehungsweise durchgeführt werden.

Bei der insbesondere elektronischen Patientenwarneinrichtung handelt es sich um eine solche Einrichtung beziehungsweise Vorrichtung, um Bildgebungssysteme mit einer einfachen Möglichkeit auszugestalten, mit welcher Patienten des Bildgebungssystems bei einer Notsituation auf einfachste Art und Weise Hilfe bekommen können. Hierzu wird mit der Signalerzeugungseinheit das zumindest eine akustische Warnsignal oder mehrere akustische Warnsignale erzeugt. Das Erzeugen des Warnsignals kann durch den Patienten initiiert beziehungsweise ausgelöst werden. Hierzu weist die Patientenwarneinrichtung das Betätigungselement auf, mittels welchem der Patient die Warnung initiieren kann. Durch das Betätigen des Betätigungselements, welches erfasst werden kann, kann die Erzeugung und insbesondere die Aussendung des akustischen Signals initiiert und insbesondere durchgeführt werden.

Die Auswerteeinheit kann beispielsweise auch als Signaldetektor bezeichnet werden, welche einen entsprechenden Alarm-Ausgang aufweisen kann. Mit diesem Alarm-Ausgang kann anschließend beispielsweise das Bedienpersonal entsprechend gewarnt werden.

Bei dem Betätigungselement handelt es sich insbesondere um ein kompaktes, tragbares Element. Insbesondere weist das Betätigungselement keine drahtgebundenen Verbindungen zu der Patientenwarneinrichtung oder zu dem Bildgebungssystem auf. Somit kann das Betätigungselement kompakt von dem Patienten in der Hand, insbesondere während eines Untersuchungsvorgangs mit dem Bildgebungssystem, gehalten werden. Des Weiteren weist die Patientenwarneinrichtung die Signalerfassungseinheit auf. Bei der Signalerfassungseinheit handelt es sich um eine solche Erfassungseinheit, um insbesondere akustische Signale erfassen zu können. Die Signalerfassungseinheit kann so ausgestaltet sein, dass sie in der Umgebung des Bildgebungssystems angeordnet werden kann. Hierbei kann drauf geachtet werden, dass das akustische Signal mit ausrechender SNR erfasst werden kann. Die Signalerfassungseinheit kann kommunikationstechnisch mit der elektronischen Auswerteeinheit verbunden beziehungsweise gekoppelt sein, sodass das empfangene akustische Warnsignal der elektronischen Auswerteeinheit, bei welchem es sich um eine elektronische Recheneinheit handeln kann, übermittelt werden kann. Diese Übermittlung kann drahtlos oder drahtgebunden erfolgen. Auf Basis des empfangenen Signals erfolgt eine signaltechnische Auswertung in der elektronischen Auswerteeinheit, sodass auf Basis des akustischen Warnsignals systemseitig erkannt werden kann, dass ein Warnhinweis durch den Patienten ausgelöst beziehungsweise initiiert wurde.

In einem Ausführungsbeispiel des weiteren Aspekts ist vorgesehen, dass die Signalerzeugungseinheit im Betätigungselement integriert ist. Da während des Untersuchungsvorgangs des Patienten mithilfe des Bildgebungssystems, bei welchem es sich um ein MRT-System handeln kann, eine kompakte Ausgestaltung hinsichtlich der Betätigung, Signalerzeugung und Signalaussendung vorteilhaft ist, kann die Signalerzeugungseinheit direkt in dem Betätigungselement integriert sein Beispielsweise kann das Betätigungselement so ausgestaltet sein, dass die Komponenten zur Signalerzeugung und zur Signalaussendung in dem Betätigungselement integriert sind. Hierzu können in einem gemeinsamen Gehäuse beziehungsweise einer gemeinsamen Hülle sowohl die Signalerzeugungseinheit als auch die Komponenten des Betätigungselements integriert sein.

Beispielsweise kann das Betätigungselement als "Quetschball" ausgebildet sein, sodass hierbei durch Quetschen dieses Quetschballs die Betätigung erfolgen kann und durch diese Betätigung kann beispielsweise im Inneren des Quetschballs die Signalerzeugung und insbesondere die Signalaussendung initiiert beziehungsweise hervorgerufen werden. Optional kann das Betätigungselement eine kleine, kompakte Einheit sein, welche mit einem sehr kurzen Schlauch am "Quetschball" angehängt werden kann.

In einem Ausführungsbeispiel des weiteren Aspekts ist vorgesehen, dass die Signalerzeugungseinheit ausgebildet ist, dass zumindest eine akustische Signal auf Basis eines elektrischen Funktionsprinzips zu erzeugen, wobei die Signalerzeugungseinheit als elektrisch angesteuerte Akustik-Signalgeber oder als Lautsprecher ausgebildet ist. Insbesondere kann die Signalerzeugungseinheit ausgebildet sein, das akustische Warnsignal als Ultraschallsignal zu erzeugen. Hierzu kann ein elektrisches Funktionsprinzip verwendet werden. Mit anderen Worten ausgedrückt wird das Ultraschallsignal auf Basis einer elektrischen Signalerzeugung erzeugt. Hierzu kann die Signalerzeugungseinheit als elektrisch angesteuerter Akustik-Signalgeber, also ein Ferroelektrischer Lautsprecher, ausgebildet sein. Optional kann hier die Signalerzeugung mittels einer Piezo-elektrischen Signalerzeugungseinheit durchgeführt werden. Hierbei kann vor allem ein Piezoelektrischer Schallwandler verwendet werden.

Die Signalerzeugungseinheit, welche beispielsweise als Lautsprecher ausgebildet ist, kann in der Hülle beziehungsweise das Gehäuse des Betätigungselements integriert sein.

In einem Ausführungsbeispiel des weiteren Aspekts ist vorgesehen, dass die Signalerzeugungseinheit ein Sicherheitsfunktionselement aufweist, wobei das Sicherheitsfunktionselement ausgebildet ist, eine Funktionsfähigkeit der Signalerzeugungseinheit automatisch zu überprüfen. Da in dieser Ausgestaltung die Signalerzeugungseinheit elektrisch basierend die Signalerzeugung vornimmt, ist eine elektrische Energieversorgung vonnöten. Diese kann beispielsweise durch eine Batterie realisiert werden. Mithilfe des Sicherheitsfunktionselements kann beispielsweise ein Batteriestatus der Batterie überprüft werden. Bevor die Funktionsfähigkeit der Batterie beeinträchtigt ist, kann eine entsprechende Warnung ausgegeben werden, Somit kann hier bereits frühzeitig eine entsprechende Warnung ausgegeben werden, dass die Signalerzeugungseinheit zu überprüfen ist.

Beispielsweise denkbar ist, dass bei einem zu niedrigem Batterieladezustand oder einem sonstigen Defekt der Signalerzeugungseinheit ein spezielles Signal ausgesendet wird, um beispielsweise mit der Signalerfassungseinheit zusätzlich erkennen zu können, dass ein fehlerhafter Zustand der Batterie vorliegt.

In einem Ausführungsbeispiel des weiteren Aspekts ist vorgesehen, dass die Signalerzeugungseinheit ausgebildet ist, dass zumindest ein akustisches Signal auf Basis eines mechanischen Funktionsprinzips zu erzeugen, wobei die Signalerzeugungseinheit einen mit einem kompressiblen Schallübertragungsmedium aufweisenden Behälter aufweist, wobei die Signalerzeugungseinheit mit dem Betätigungselement derart gekoppelt ist, dass auf Basis der Betätigung des Betätigungselements Druckschwankungen im dem Behälter hervorgerufen werden, wodurch das zumindest eine akustische Signal erzeugbar ist. Somit kann hier entgegen der elektrischen Signalerzeugung eine mechanische Signalerzeugung vorgenommen werden. Hierbei ergibt sich der Vorteil, dass keine Energieversorgung hinsichtlich der elektrischen Signalerzeugung notwendig ist. Somit kann beispielsweise eine Ladeinfrastruktur entfallen und es ist auch nicht erforderlich eine Batterie, wie beispielswiese einen Akkumulator, in regelmäßigen Abständen zu laden und/oder zu tauschen. Die Verwendung des mechanischen Signalerzeugungsprinzips ist des Weiteren kostengünstig, da eine minimierte Infrastruktur erforderlich ist. Ein weiterer Vorteil ist, dass durch den Wegfall von zusätzlicher Elektronik hinsichtlich der Signalerzeugung EMC-HF-Rauschteilprobleme vermieden werden können, die Bildartefakte beispielsweise in der MRT-Messung hervorrufen können. Aufgrund der nicht benötigten zusätzlichen Elektronik kann auch eine diesbezüglich spezielle Abschirmung vermieden werden, was ebenfalls Bildartefakte oder Erwärmungserscheinungen durch Anregen von Wirbelströmen verursachen könnte.

In dieser Ausgestaltung kann die Signalerzeugungseinheit ebenfalls ausgebildet sein, ein Ultraschallsignal als akustisches Signal rein mechanisch zu erzeugen. Dies kann beispielsweise durch die Verwendung eines Druckballs realisiert werden.

Des Weiteren bietet die mechanische Signalerzeugung den Vorteil, dass aufgrund des Wegfalls der Elektronik hinsichtlich der elektronischen Signalerzeugung keine speziellen behördlichen Genehmigungen (bspw. bzgl. Funkzulassung) notwendig sind.

Um eine sichere mechanische Signalerzeugung durchführen zu können, kann des Weiteren ein zusätzlicher Mechanismus vorgesehen sein, dass auch bei einer leichten beziehungsweise nur kurzen Betätigung dennoch ein ausreichender Mindestdruck hinsichtlich des Behälters vorliegt. Dies kann beispielsweise durch Druckventile realisiert werden. Somit kann erreicht werden, dass das Schallübertragungsmedium, bei welchem es sich beispielsweise um Luft handeln kann, eine ausreichende Druckschwankung erfährt, wodurch eine Schallerzeugung und insbesondere Schallausbreitung hervorgerufen werden kann.

In einem Ausführungsbeispiel des weiteren Aspekts ist vorgesehen, dass die Signalerfassungseinheit zumindest ein Empfangselement, insbesondere ein Mikrofon, aufweist. Beispielsweise kann das Empfangselement als ultraschallfähiges Mikrophon ausgebildet sein, um insbesondere Ultraschallsignale als akustische Signale empfangen zu können.

Des Weiteren ist denkbar, dass mehrere solcher Empfangselemente vorgesehen sind. Das Empfangselement kann beispielsweise ein bereits bestehendes Mikrophon im Untersuchungsraum des Bildgebungssystems sein, sodass hier dieses zweckentfremdet werden kann, wodurch der Einsatz zusätzlicher Bauteile vermieden werden kann. Insbesondere kann die Signalerfassungseinheit mehrere Empfangselemente aufweisen, welche in der Umgebung des Bildgebungssystems integriert beziehungsweise angeordnet werden können, um eine zuverlässige Signaldetektion vornehmen zu können.

In einer weiteren Ausgestaltung des weiteren Aspekts ist vorgesehen, dass die Signalerfassungseinheit und elektronische Auswerteeinheit jeweils eine Schnittstelleneinheit aufweisen, wobei die Schnittstelleneinheiten ausgebildet sind, die Signalerfassungseinheit und die elektronische Auswerteeinheit mit einer Steuerung des Bildgebungssystems zu koppeln. Um vor allem bei der Erkennung des Warnhinweises eine entsprechende Hilfsmaßnahme dem Patienten bereitstellen zu können, ist eine entsprechende Informationsübertragung hinsichtlich des erkannten Warnhinweises an das Bildgebungssystem und/oder an das Bedienpersonal des Bildgebungssystems notwendig. Diese Informationsübertragung kann optional über die Schnittstelleneinheiten erfolgen.

In einem Ausführungsbeispiel des weiteren Aspekts ist vorgesehen, dass das Betätigungselement derart ausgestaltet ist, dass es in einer Hand des Patienten während des Untersuchungsvorgangs tragbar ist. Das Betätigungselement ist speziell kompakt ausgestaltet, sodass es während des Untersuchungsvorgangs durch den Patienten getragen beziehungsweise in der Hand gehalten werden kann. Mit anderen Worten ausgedrückt kann das Betätigungselement so ausgestaltet sein, dass es mit einer Hand des Patienten problemlos umgriffen werden kann. Somit kann der Patient das Betätigungselement während des Untersuchungsvorgangs in der Hand halten und bei einer Gefahrensituation entsprechend leicht die Betätigung ausüben.

Ein weiterer Aspekt der Erfindung betrifft ein Bildgebungssystem mit einer Bildgebungsmodalität und einer Patientenwarneinrichtung nach dem vorherigen Aspekt oder einer vorteilhaften Weiterbildung davon.

Insbesondere kann es sich bei dem Bildgebungssystem um ein MRT-System oder um ein CT-System (Computertomographie-System) handeln. Insbesondere dient das Bildgebungssystem dazu, um Untersuchungen an Patienten vornehmen zu können.

Ausführungen eines Aspekts sind als vorteilhafte Ausführungen des anderen Aspekts und umgekehrt anzusehen.

Zu der Erfindung gehören auch Weiterbildungen der erfindungsgemäßen Patientenwarneinrichtung und des erfindungsgemäßen Bildgebungssystems, die Merkmale aufweisen, wie sie bereits im Zusammenhang mit den Weiterbildungen des erfindungsgemäßen Verfahrens beschrieben worden sind. Aus diesem Grund sind die entsprechenden Weiterbildungen der erfindungsgemäßen Patientenwarneinrichtung und des erfindungsgemäßen Bildgebungssystems hier nicht noch einmal beschrieben.

Die Erfindung umfasst auch die Kombinationen der Merkmale der beschriebenen Ausführungsformen.

Bei den im Folgenden erläuterten Ausführungsbeispielen handelt es sich um bevorzugte Ausführungsbeispiele der Erfindung. Bei den Ausführungsbeispielen stellen die beschriebenen Komponenten jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsbeispiele auch durch weitere der bereits beschriebenen Merkmale der Erfindung ergänzbar.

Die vorliegende Erfindung wird nun anhand der beigefügten Zeichnungen näher erläutert, in denen zeigen:
- FIG 1: eine schematische Ansicht eines Bildgebungssystems, welches mit einer Patientenwarneinrichtung ausgestattet ist;
- FIG 2: eine schematische Ansicht einer Ausführungsform eines Betätigungselements und einer Signalerzeugungseinheit der Patientenwarneinrichtung aus FIG 1;
- FIG 3: eine weitere mögliche Ausgestaltungsform der Signalerzeugungseinheit und des Betätigungselements der Patientenwarneinrichtung aus FIG 1; und
- FIG 4: einen schematischen Ablauf zur Erkennung einer durch den Patienten ausgelösten Warnung beziehungsweise eines Warnhinweises durch signaltechnische Auswertung.

Die nachfolgend näher geschilderten Ausführungsbeispiele stellen bevorzugte Ausführungsformen der vorliegenden Erfindung dar.

In den Figuren sind funktionsgleiche Elemente mit denselben Bezugszeichen versehen.

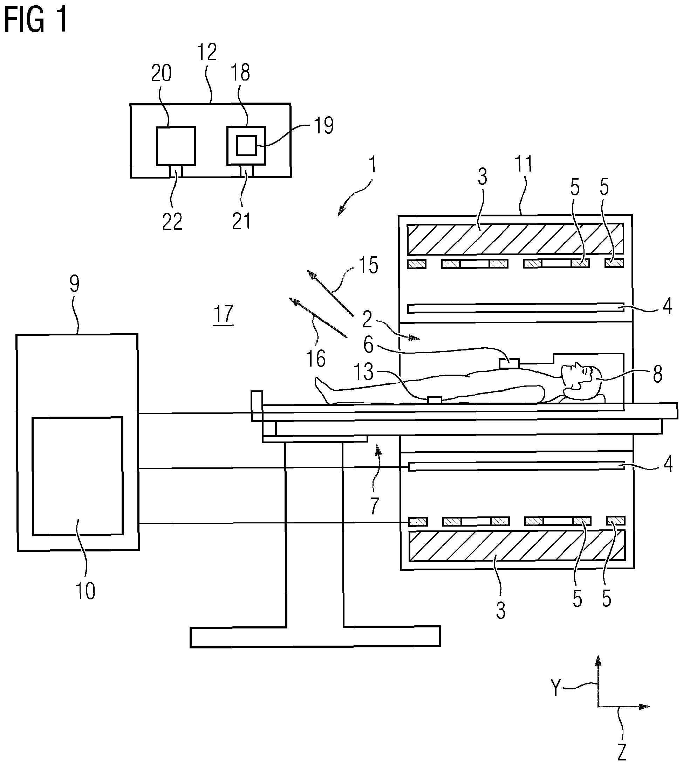

FIG 1 zeigt eine schematische Darstellung einer beispielhaften Ausführungsform eines Bildgebungssystems 1, welches als (MRT-) Systems ausgebildet sein kann (auch als Magnetresonanzsystem bezeichnet).

Das MRT-System als Bildgebungssystem 1 umfasst eine Magneteinheit mit einem Feldmagneten 3, der ein statisches Magnetfeld zur Ausrichtung von Kernspins eines Objekts 8, zum Beispiel eines Patienten, in einem Bildgebungsbereich erzeugt. Der Bildgebungsbereich ist durch ein äußerst homogenes statisches Magnetfeld gekennzeichnet, wobei sich die Homogenität insbesondere auf die Magnetfeldstärke beziehungsweise deren Amplitude bezieht. Der Bildgebungsbereich befindet sich in einem Patiententunnel 2, der sich in einer Längsrichtung Z durch die Magneteinheit erstreckt. Der Feldmagnet 3 kann beispielsweise ein supraleitender Magnet sein, der Magnetfelder mit einer magnetischen Flussdichte von bis zu 3 T oder mehr erzeugen kann. Für geringere Feldstärken können jedoch auch Permanentmagnete oder Elektromagnete mit normalleitenden Spulen verwendet werden. Ein Patiententisch beziehungsweise Untersuchungstisch 7 kann innerhalb des Patiententunnels 2 beweglich sein.

Weiterhin umfasst die Magneteinheit zumindest eine Gradientenspule 5. Ebenso denkbar ist, dass die Gradientenspule 5 aus einer Anordnung von mehreren Teil-Gradientenspulen besteht. Die Gradientenspule 5 dient dazu, dem statischen Magnetfeld Gradientenfelder, also ortsabhängige Magnetfelder, in den drei Raumrichtungen zur räumlichen Differenzierung der abgetasteten Bildbereiche im Bildgebungsbereich zu überlagern. Die Gradientenspule 5 kann zum Beispiel als Spule aus normalleitenden Drähten ausgebildet sein, die zum Beispiel zueinander orthogonale Felder oder Feldgradienten im Bildgebungsbereich erzeugen können.

Die Magneteinheit umfasst eine Sendespulenanordnung, die beispielsweise eine Körperspule 4 (auch als Ganzkörperspule oder Body Coil bezeichnet) als Sendeantenne umfassen kann, die dazu ausgebildet ist, ein Hochfrequenzsignal bzw. Anregungssignal in den Bildgebungsbereich abzustrahlen. Die Körperspule 4 kann daher als HF-Sendespulenanordnung des MRT-Systems verstanden werden oder als Teil der HF-Sendespulenanordnung. Die Körperspule 4 kann in einigen Ausführungsformen auch dazu verwendet werden, resonante MR-Signale zu empfangen, die von dem Objekt 8 ausgesendet werden. In diesem Fall kann die Körperspule 4 auch als Teil einer Signalerfassungsvorrichtung des MRT-Systems betrachtet werden. Optional umfasst die Signalerfassungsvorrichtung eine lokale Spule 6 bzw. Lokalspule, die in unmittelbarer Nähe des Objekts 8, zum Beispiel an dem Objekt 8 oder in dem Patiententisch 7, angeordnet sein kann. Die lokale Spule 6 kann alternativ oder zusätzlich zur Körperspule 4 als Empfangsspule beziehungsweise Empfangsantenne dienen.

Das MRT-System umfasst auch ein Steuer- und Rechensystem 9. Das Steuer- und Rechensystem 9 kann eine Sende-Empfangssteuereinheit 10 umfassen, die mit der Körperspule 4, der Gradientenspule 5 und/oder der lokalen Spule 6 verbunden ist. In Abhängigkeit von den erfassten MR-Signalen kann die Sende-Empfangssteuereinheit 10, die einen Analog-Digital-Wandler, ADC (englisch: "analog-to-digital converter") umfassen kann, entsprechende MR-Daten, insbesondere im k-Raum, erzeugen. Die Sende-Empfangs-Steuereinheit bzw. Sende-Empfangssteuereinheit 10 ist gegebenenfalls auch mit der Körperspule 4 verbunden und steuert diese zur Erzeugung von HF-Impulsen, wie Anregungsimpulsen und/oder Refokussierungsimpulsen, an. Weiterhin kann die Sende-Empfangssteuereinheit 10 des Steuer- und Rechensystems 9 auch mit der Gradientenspule 5 verbunden sein und diese steuern, um Schichtselektionsgradienten, Gradienten für die Frequenz- und/oder Phasencodierung und/oder Auslesegradienten zu schalten.

Beispielsweise weist das MRT-System eine Bildgebungsmodalität 11 auf. Die Bildgebungsmodalität 11 kann zumindest die Magneteinheit, den Patiententisch 7 und den Patiententunnel 2 aufweisen.

Wenn sich der Patient 8 auf den Patiententisch 7 innerhalb der Bildgebungsmodalität 11 befindet und ein dementsprechender Untersuchungsvorgang durchgeführt wird, kann es vorkommen, dass aufgrund des begrenzten Platzbereichs der Patient 8 in Panik gerät, beispielsweise aufgrund einer Klaustrophobie. Anderenfalls kann es vorkommen, dass aufgrund eines medizinischen Notfalls der Patient 8 sich unwohl fühlt oder sogar gesundheitliche Probleme aufweist. Das Bedienpersonal, also das medizinische Personal, befindet sich während des Untersuchungsvorgangs in einem zum Untersuchungsraum, in welchem sich die Bildgebungsmodalität 11 befindet, entfernten Bereich. Daher kann es vorkommen, dass das Bedienpersonal keinen Sichtkontakt zum Patienten 8 hat, sodass bei einem Notfall des Patienten 8 das Bedienpersonal verspätet reagieren kann. Diesbezüglich gibt es bei heutigen Bildgebungssystemen 1 pneumatische Systeme. Hierbei weist ein solches pneumatisches System einen drückbaren Quetschball auf, welcher über einen langen Schlauch hinsichtlich des pneumatischen Systems mit dem Bildgebungssystem verbunden ist.

Wie bereits eingangs erwähnt ist dieses pneumatische System bauraumbeanspruchend und auch für das Bedienpersonal gefährlich. Durch den langen Schlauch, welcher auf dem Boden im Bereich der Bildgebungsmodalität 11 herumliegen kann, können Stolperstellen entstehen. Dies ist für den Patienten als auch für das Bedienpersonal gefährlich. Um hier eine effizientere und platzsparende Möglichkeit bereitzustellen, kommt die vorliegende Erfindung zum Einsatz. Anstelle des pneumatischen Systems und der festen Schlauchverbindung erfolgt das Ausgeben eines Hilferufs beziehungsweise einer Warnung auf Basis akustischer Signale und einer entsprechenden signaltechnischen Auswertung. Hierzu ist erfindungsgemäß eine Patientenwarneinrichtung 12 vorgesehen.

Bei dieser elektronischen Patientenwarneinrichtung 12 handelt es sich insbesondere um ein Notfallsystem, Notfalleinrichtung oder Warneinrichtung, mit welcher dem Patienten während eines Untersuchungsvorgangs im Bildgebungssystem 1 die Möglichkeit bereitgestellt wird im Falle eines Notfalls eine entsprechende Warnung in Form eines Warnhinweises auszugeben. Die Patientenwarneinrichtung 12 kann aus mehreren einzelnen Teilen ausgebildet sein, welche zumindest teilweise mit dem Bildgebungssystem 1 gekoppelt beziehungsweise kommunikativ vernetzt werden können.

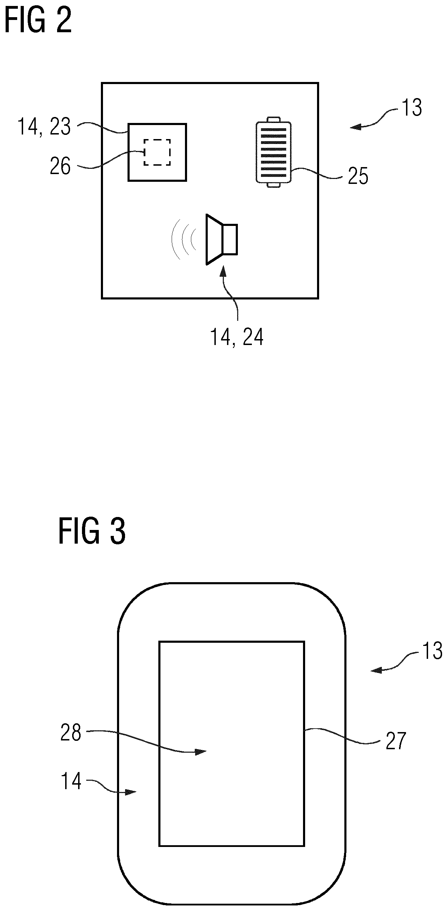

Insbesondere weist die Patientenwarneinrichtung 12 ein Betätigungselement 13 auf. Dieses Betätigungselement 13 kann so ausgebildet sein, dass es insbesondere in einer Hand des Patienten 8 während des Untersuchungsvorgangs getragen beziehungsweise gehalten werden kann. Dadurch kann der Patient 8 eine Warnung beziehungsweise einen Notruf initiieren. Das Betätigungselement 13 ist insbesondere so ausgestaltet, dass durch eine Betätigung des Patienten 8 eine Signalerzeugung initiiert und insbesondere aktiviert werden kann. Hierzu weist des Weiteren die Patientenwarneinrichtung 12 eine Signalerzeugungseinheit 14 (vergleiche Figur 2) auf. Mit dieser Signalerzeugungseinheit 14 kann zumindest ein akustisches Signal 15 erzeugt werden. Des Weiteren können mehrere solcher Signale erzeugt werden, wie beispielsweise das weitere akustische Signal 16. Des Weiteren kann die Signalerzeugungseinheit 14 so ausgebildet sein, dass das erzeugte, akustische Warnsignal 15 in eine Umgebung 17 des Bildgebungssystems 1 ausgesendet werden kann. Um nun die von dem Patienten 8 initiierte Warnung entsprechend erkennen zu können, weist die Patientenwarneinrichtung 12 zumindest eine Signalerfassungseinheit 18 auf. Diese Signalerfassungseinheit 18 kann vor allem ein Empfangselement 19 oder mehrere solcher Empfangselemente aufweisen. Vor allem handelt es sich bei einem solchen Empfangselement 19 um ein Mikrofon. Hierzu können entweder bereits vorhandene Mikrofone hinsichtlich des Bildgebungssystems 1 oder ein separates Mikrofon verwendet werden. Mit Hilfe des Mikrofons kann also das ausgesendete akustische Warnsignal 15 empfangen werden. Um dies entsprechend auswerten zu können, weist die Patientenwarneinrichtung 12 des Weiteren eine elektronische Auswerteeinheit 20 auf, mit welcher eine entsprechende Signalverarbeitung beziehungsweise Signalauswertung beziehungsweise Signaldetektion durchgeführt werden kann. Hierbei kann vor allem eine Signalauswertung bezüglich eines durch den Patienten 8 während des Untersuchungsvorgangs initiierten Warnhinweises beziehungsweise Notrufes beziehungsweise Notfalls ausgewertet werden. Auf Basis dieser Signalauswertung kann eine entsprechende Erkennung oder Detektion des Warnhinweises hinsichtlich des Patienten 8 durchgeführt werden, um entsprechende Sicherheitsmaßnamen durch das Bildgebungssystem 1 und/oder durch das Bedienpersonal ausführen zu können.

Hierbei kann entweder eine Warnung an das Bedienpersonal ausgegeben werden oder auf Basis des erkannten Warnhinweises kann systemseitig ein entsprechendes Steuersignal generiert werden, um vor allem Eingriff in die Funktion beziehungsweise Betrieb des Bildgebungssystems 1 vorzunehmen.

Um insbesondere solche Steuersignale an das Bildgebungssystem 1 weiterleiten zu können, kann die Signalerfassungseinheit 18 als auch die Auswerteeinheit 20 jeweils eine Schnittstelleneinheit 21, 22 aufweisen. Diese ermöglichen eine Kopplung beziehungsweise kommunikationstechnische Verbindung mit dem Steuer- und Rechensystem 9. Beispielsweise ist denkbar, dass das akustische Signal 15 als Ultraschallsignal ausgesendet wird, sodass vor allem die Signalerfassungseinheit 18 zur Erfassung von Ultraschallsignalen ausgestaltet sein muss.

Bei der Verwendung von dem Mikrofon 19, welches separat zu dem Bildgebungssystem 1 ausgebildet ist, liegt der Vorteil darin, dass eine Entkopplung hinsichtlich der Funktion des Bildgebungssystems 1 gewährleistet ist und das Mikrofon 19 ausschließlich hinsichtlich der Alarmdetektion, also der Erkennung des Warnhinweises verwendet werden kann.

Die Erkennung kann beispielsweise analog durchgeführt werden oder anhand eines Schwellwerterkennens mit Filter und optionaler PLL-Verriegelung auf Ultraschallsignale bei ein oder mehreren Frequenzen. Ebenso denkbar ist, dass die Signalerfassungseinheit 18 in Kombination mit der Auswerteeinheit 20 das erfasste akustische Signal 15 digitalisiert, um das akustische Signal 15 hinsichtlich des ausgelösten Alarms des Patienten 8 identifizieren zu können. Dies ist vor allem dann von Vorteil, wenn aufgrund des laufenden Messvorgangs mittels des Bildgebungssystems 1 es zu niedrigen SNR-Werten kommt. Beispielsweise wäre es denkbar, dass über die Schnittstellen 21, 22 Zustandsinformationen hinsichtlich des Zustands der Patientenwarneinrichtung 12 an das Bildgebungssystem 1 übermittelt werden. Sollte sich die Patientenwarneinrichtung 12 nicht in einem funktionsfähigen Zustand befindet, so können entsprechende Vorkehrungen vorgenommen werden.

Beispielsweise kann mittels der Signalerzeugungseinheit 14 ein Zustand einer Batterie oder eines sonstigen Geräts der Signalerzeugungseinheit, insbesondere des Bedienelements, übermittelt werden. Somit kann eine Aktualisierung vorgenommen werden, sodass beispielsweise ein Algorithmus hinsichtlich der Signalauswertung verbessert werden kann. Beispielsweise kann das beim Betätigen ausgesendete akustische Warnsignal 15 auch zusätzliche Informationen, wie eine Information eines zu niedrigen Ladezustands der Batterie, enthalten. Dadurch kann das Bedienpersonal rechtzeitig gewarnt werden, dass ab jetzt nur noch für eine gewisse Zeit Warnungen signaltechnisch ausgesendet werden können. Sollte es beispielsweise zu Veränderungen gekommen sein, so könnte eine Anpassung hinsichtlich Frequenzen oder des Modulationsverfahrens vorgenommen werden.

In der Figur 2 ist eine mögliche Ausgestaltung des Betätigungselements 13 aus der Figur 1 gezeigt. Optional kann die Signalerzeugungseinheit 14 in dem Betätigungselement 13 integriert werden, sodass unmittelbar bei einem Betätigen des Betätigungselements 13 eine entsprechende Wirkung auf die Signalerzeugungseinheit 14 ausgeübt werden kann.

In der Ausgestaltung gemäß FIG 2 erfolgt die Signalerzeugung hinsichtlich des akustischen Signals 15 elektrisch, elektronisch und/oder elektromechanisch. Mit anderen Worten ausgedrückt erfolgt hier durch das Betätigen des Betätigungselements 13 eine elektrische Signalerzeugung. Hierzu kann in dieser Ausgestaltungsform die Signalerzeugungseinheit 14 als elektrisch angesteuerter Akustik-Signalgeber 23 ausgestaltet sein. Mit anderen Worten ausgedrückt kann hier auf Basis eines piezoelektrischen Effekts eine Signal-, insbesondere Schallerzeugung, durchgeführt werden. Hierbei kann dieser elektrisch angesteuert Akustik-Signalgeber als ferroelektrischer Lautsprecher beispielsweise ausgebildet sein. Im einfachsten Fall kann des Weiteren die Signalerzeugungseinheit 14 als Lautsprecher 24 ausgestaltet sein. Durch das Betätigen des Betätigungselements 13 kann insbesondere ein elektrisches Signal zu der Signalerzeugungseinheit 14 übermittelt werden, sodass wiederum die Signalerzeugung durchgeführt werden kann. In dieser Ausgestaltung kann des Weiteren eine Batterie 25 in dem Betätigungselement 13 integriert sein, um die elektrische Signalerzeugung durchführen zu können. In diesem Fall kann des Weiteren die Signalerzeugungseinheit 14 ein Sicherheitsfunktionselement 26 aufweisen, um die jeweilige Funktionsfähigkeit der Signalerzeugungseinheit 14 überprüfen zu können. Hierbei kann vor allem der Batteriezustand der Batterie, insbesondere kontinuierlich, überprüft und je nachdem kann wiederum an die Auswerteeinheit 20 über die Signalerfassungseinheit 18 ein entsprechendes Signal übermittelt werden.

In der FIG 3 ist eine weitere mögliche Ausgestaltung hinsichtlich der Signalerzeugung dargestellt. Um die Signalerzeugung noch einfacher zu gestalten und die Signalerzeugung unabhängig von magnetischen Feldern hinsichtlich des Bildgebungssystems 1 durchzuführen, kann die Signalerzeugung mechanisch durchgeführt werden. Hierbei kann wiederum die Signalerzeugungseinheit 14 in dem Betätigungselement 13 integriert werden. Mit anderen Worten ausgedrückt können die beiden Einheiten 13, 14 ein gemeinsames Gehäuse beziehungsweise eine gemeinsame Geometrie aufweisen. In dieser Ausgestaltung wird ein mechanisches Wirkprinzip beziehungsweise Funktionsprinzip für die Signalerzeugung verwendet. Hierbei kann die Signalerzeugungseinheit 14 einen Behälter 27 aufweisen, in welchem ein kompressibles Schallübertragungsmedium 28, insbesondere Luft, ausgebildet ist. Der Behälter 27 steht im direkten Kontakt mit dem Betätigungselement 13, sodass beim Betätigen des Betätigungselements 13 eine Krafteinwirkung beziehungsweise eine Druckeinwirkung auf den Behälter 27 ausgeübt wird, wodurch eine Druckschwankung hervorgerufen wird, welche wiederum zur Schallerzeugung und somit zur Erzeugung des akustischen Signals 15 verwendet werden kann. Mit anderen Worten ausgedrückt kann der Patient 8 durch Drücken des Betätigungselements 13 mit seiner Hand die Druckschwankungen hervorrufen, sodass dadurch eine Schallausbreitung hervorgerufen wird und dadurch wiederum das akustische Signal 15 erzeugt wird.

Des Weiteren kann die Patientenwarneinrichtung 12 nicht nur während des Untersuchungsvorgangs verwendet werden, sondern auch bei der Vorbereitung als auch bei der Nachbereitung. Mit anderen Worten ausgedrückt kann beispielsweise beim Einfahren des Patiententisches 7 in den Patiententunnel dem Patienten 8 mithilfe des Betätigungselements 13 und der Signalerzeugungseinheit 14 die Möglichkeit gegeben werden, bei einer Kollision, wie einer Quetschung, eine Warnung beziehungsweise den Warnhinweis auszugeben. Dies kann wiederum direkt dem Steuer- und Rechensystem 9 bereitgestellt werden. Bei der Aussendung von Ultraschallsignalen kann durch die Anordnung mehrerer Mikrofone in der Umgebung 17 auf Basis von einer Time-of-Flight-Bestimmung und somit einer Triangulation eine Position des Betätigungselements 13 und somit des Patienten 8 ermittelt werden, was wiederum für die Positionierung beziehungsweise Lokalisierung des Patienten 8 verwendet werden kann.

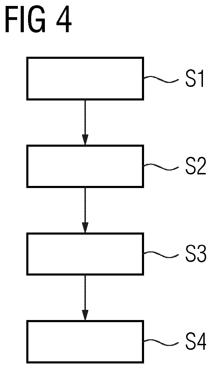

In der FIG 4 ist ein beispielhafter Ablauf hinsichtlich der Erkennung des Warnsignals betreffend des Patienten 8 dargestellt.

In einem optionalen Schritt S1 kann der Patient 8 für den Untersuchungsvorgang mit dem Bildgebungssystem 1 entsprechend vorbereitet werden. Hierbei kann dem Patienten 8 das Betätigungselement 13 entsprechend bereitgestellt werden, sodass der Patient 8 dies während des Untersuchungsvorgangs in seiner Hand halten kann.

In einem optionalen Schritt S2 kann nun während des Untersuchungsvorgangs im Falle einer Notsituation oder eines klaustrophobischen Zustands der Patient 8 das Betätigungselement 13 betätigen. Dadurch kann vor allem die Signalerzeugungseinheit 14 entsprechend aktiviert werden, sodass entweder auf Basis einer mechanischen oder elektrischen Signalerzeugung das akustische Signal 15 erzeugt werden kann.

In einem optionalen Schritt S3 kann nun die Signalerzeugung durchgeführt werden. Hierbei kann das akustische Signal in einem Hörfrequenzbereich oder in einem Ultraschallfrequenzbereich erzeugt werden. Besonders vorteilhaft ist die Aussendung des akustischen Signals 15 als Ultraschallsignal, da dieses weniger störanfällig hinsichtlich des Bildgebungssystems 1 ist. Je nach Situation, insbesondere je nach Anwendungsgebiet, kann eine Frequenz des akustischen Signals 15 auf Basis des durch das Bildgebungssystem 1 hervorgerufenen Störsignals angepasst werden. Ebenso denkbar ist, dass eine Modulation hinsichtlich des akustischen Signals 15 durchgeführt wird. Hierbei kann das akustische Signal 15 derart moduliert werden, dass eine verbesserte Detektion dieses Signals durchgeführt werden kann, sodass fehlerhafte Interpretationen möglichst ausgeschlossen werden können.

Zur besseren Erkennung beziehungsweise Identifizierung hinsichtlich der Warnung beziehungsweise Alarmierung hinsichtlich des Patienten 8 kann neben dem akustischen Signal 15 auch das weitere akustische Signal 16 oder mehrere solche Signale ausgesendet werden, sodass erst bei Erkennung beziehungsweise Detektion beider Signale 15, 16 die Auswertung hinsichtlich des Warnhinweises beziehungsweise der Warnung vorgenommen wird.

In einem optionalen Schritt S4 kann das akustische Signal 15 und/oder das akustische Signal 16 entsprechend ausgewertet werden, um daraufhin den Warnhinweis zu erkennen und somit die durch den Patienten 8 selbst initiierte Alarmierung aufgrund einer aktuellen Gefahrensituation während des Untersuchungsvorgangs erkennen zu können. Dementsprechend kann das Bedienpersonal des Bildgebungssystems informiert werden oder es erfolgt eine automatische Auslösung einer Sicherheitsfunktion des Bildgebungssystems 1.

Mit den Ausgestaltungen gemäß der FIG 1 bis 4 kann ein kabelloser Quetschball realisiert werden, mit welchem mittels akustischer Triggerung eine entsprechende Warnung beziehungsweise Warnhinweis des Patienten initiiert beziehungsweise ausgelöst werden kann.

## Patentansprüche

1. Verfahren zum Erkennen eines Warnhinweises eines Patienten (8), während der Patient (8) mittels eines Bildgebungssystems (1) untersucht wird, wobei
- zumindest ein akustisches Warnsignal (15), mit welchem der Warnhinweis des Patienten (8) repräsentiert wird, auf Basis einer Betätigung eines Betätigungselements (13) durch den Patienten (8) erzeugt und in eine Umgebung (17) des Bildgebungssystems (1) ausgesendet wird,
- das in die Umgebung (17) ausgesendete akustische Warnsignal (15) empfangen und einer elektronischen Auswerteeinheit (20) bereitgestellt wird,
- das empfangene und bereitgestellte akustische Warnsignal (15) durch die elektronische Auswerteeinheit (20) ausgewertet wird, und
- der Warnhinweis des Patienten (8) auf Basis des ausgewerteten akustischen Warnsignals (15) erkannt wird.

2. Verfahren nach Anspruch 1, wobei auf Basis von dem erkannten Warnhinweis eine Warnung an ein Bedienpersonal des Bildgebungssystems (1) ausgegeben wird, und/oder auf Basis von dem erkannten Warnhinweis eine Sicherheitsfunktion des Bildgebungssystems (1) automatisch aktiviert wird.

3. Verfahren nach Anspruch 1 oder 2, wobei das zumindest eine akustische Warnsignal (15) so erzeugt wird, dass es in einem Hörfrequenzbereich oder einem Ultraschallfrequenzbereich liegt.

4. Verfahren nach einem der vorhergehenden Ansprüche, wobei zumindest eine Komponente des akustischen Warnsignals (15) derart erzeugt wird, indem zumindest ein durch das Bildgebungssystem (1) hervorgerufenes Störsignal berücksichtigt wird.

5. Verfahren nach einem der vorhergehenden Ansprüche, wobei das akustische Warnsignal (15) auf Basis von einer Identifizierungsinformation bezüglich der Auswertung des empfangenen akustischen Warnsignals (15) oder auf Basis von zumindest einer Zusatzinformation moduliert wird.

6. Verfahren nach einem der vorhergehenden Ansprüche, wobei neben dem zumindest einen akustischen Warnsignal (15) zumindest ein weiteres akustisches Warnsignal (16), mit welchem der Warnhinweis des Patienten (8) ebenfalls repräsentiert wird, erzeugt und ausgesendet wird, wobei das zumindest eine akustische Warnsignal (15) und das zumindest eine weitere akustische Warnsignal (16) zueinander verschiedene Frequenzen aufweisen, und wobei das zumindest eine weitere akustische Warnsignal (16) empfangen und ebenfalls der elektronischen Auswerteeinheit (20) bereitgestellt wird.

7. Patientenwarneinrichtung (12) für ein Bildgebungssystem (1), mit
- einer Signalerzeugungseinheit (14), welche ausgebildet ist, zumindest ein akustisches Warnsignal (15) auf Basis einer Betätigung eines Bestätigungselements (13) der Patientenwarneinrichtung (12) durch einen Patienten (8) zu erzeugen und in eine Umgebung (17) des Bildgebungssystems (1) auszusenden,
- einer Signalerfassungseinheit (18), welche ausgebildet ist, das in die Umgebung ausgesendete akustische Warnsignal (15) zu empfangen und einer elektronischen Auswerteeinheit (20) der Patientenwarneinrichtung (12) bereitzustellen,
- der elektronischen Auswerteeinheit (20), welche ausgebildet ist, das empfangene und bereitgestellte akustische Warnsignal (15) bezüglich eines durch den Patienten (8) während eines mit dem Bildgebungssystem (1) am Patienten (8) durchgeführten Untersuchungsvorgangs initiierten Warnhinweises (15) auszuwerten und den Warnhinweis des Patienten (8) auf Basis des ausgewerteten akustischen Warnsignals (15) zu erkennen.

8. Patientenwarneinrichtung (12) nach Anspruch 7, wobei die Signalerzeugungseinheit (14) in dem Betätigungselement (13) integriert ist.

9. Patientenwarneinrichtung (12) nach Anspruch 7 oder 8, wobei die Signalerzeugungseinheit (14) ausgebildet ist, das zumindest eine akustische Signal (15) auf Basis eines elektrischen Funktionsprinzips zu erzeugen, wobei die Signalerzeugungseinheit (14) als elektrisch angesteuerter Akustik-Signalgeber (23) oder als Lautsprecher (24) ausgebildet ist.

10. Patientenwarneinrichtung nach Anspruch 9, wobei die Signalerzeugungseinheit (14) ein Sicherheitsfunktionselement (26) aufweist, wobei das Sicherheitsfunktionselement (14) ausgebildet ist, eine Funktionsfähigkeit der Signalerzeugungseinheit (14) automatisch zu überprüfen.

11. Patientenwarneinrichtung (12) nach Anspruch 7 oder 8, wobei die Signalerzeugungseinheit (14) ausgebildet ist, das zumindest eine akustische Signal (15) auf Basis eines mechanischen Funktionsprinzips zu erzeugen, wobei die Signalerzeugungseinheit (14) einen mit einem kompressiblen Schallübertragungsmedium (28) aufweisenden Behälter (27) aufweist, wobei die Signalerzeugungseinheit (14) mit dem Bestätigungselement (13) derart gekoppelt ist, dass auf Basis der Betätigung des Bestätigungselements (13) Druckschwankungen in dem Behälter (27) hervorgerufen werden, wodurch das zumindest eine akustische Signal (15) erzeugbar ist.

12. Patientenwarneinrichtung (12) nach einem der vorhergehenden Ansprüche 7 bis 11, wobei die Signalerfassungseinheit (18) zumindest ein Empfangselement (19), insbesondere ein Mikrofon, aufweist.

13. Patientenwarneinrichtung (12) nach einem der vorhergehenden Ansprüche 7 bis 12, wobei die Signalerfassungseinheit (18) und die elektronische Auswerteeinheit (20) jeweils eine Schnittstelleneinheit (21, 22) ausweisen, wobei die Schnittstelleneinheiten (21, 22) ausgebildet sind, die Signalerfassungseinheit (18) und die elektronische Auswerteeinheit (20) mit einer Steuerung des Bildgebungssystems (1) zu koppeln.

14. Patientenwarneinrichtung (12) nach einem der vorhergehenden Ansprüche 7 bis 13, wobei das Bestätigungselement (13) derart ausgestaltet ist, dass es in einer Hand des Patienten (8) während des Untersuchungsvorgangs tragbar ist.

15. Bildgebungssystem (1) mit einer Bildgebungsmodalität (11) und einer Patientenwarneinrichtung (12) nach einem der vorhergehenden Ansprüche 7 bis 14.
